(19) Europäisches Patentamt
European Patent Office
Office européen des brevets

(11) Numéro de publication: **0 214 058 B1**

(12) **FASCICULE DE BREVET EUROPEEN**

(45) Date de publication de fascicule du brevet: **06.05.92**

(51) Int. Cl.5: **A61K 31/415**

(21) Numéro de dépôt: **86401917.9**

(22) Date de dépôt: **01.09.86**

(54) **Composition pharmaceutique contenant des dérivés de l'histamine.**

(30) Priorité: **02.09.85 FR 8513024**

(43) Date de publication de la demande: **11.03.87 Bulletin 87/11**

(45) Mention de la délivrance du brevet: **06.05.92 Bulletin 92/19**

(84) Etats contractants désignés: **BE CH DE FR GB IT LI LU**

(56) Documents cités:
**GB-A- 1 192 954**
**US-A- 3 988 466**
**US-A- 4 100 349**

**JOURNAL OF MEDICINAL CHEMISTRY, vol. 16, no. 6, 1973, pp 616-620; C.R. GANELLIN et al.: "Conformation of histamine derivatives. 2. Molecular orbital calculations of preferred conformations in relation to dual receptor activity"**

**JOURNAL OF MEDICINAL CHEMISTRY, vol. 13, janvier 1970, pp 33-35; A. BURGER et al.: "2-(4-Imidazolyl)-cyclopropylamine"**

(73) Titulaire: **INSTITUT NATIONAL DE LA SANTE ET DE LA RECHERCHE MEDICALE (INSERM)**
**101, rue de Tolbiac**
**F-75654 Paris Cédex 13(FR)**

(72) Inventeur: **Arrang, Jean-Michel Bâtiment 11 Rés. du Château Courcelles 160 Av. du Gal Leclerc**
**F-91190 Gif/Yvette(FR)**
Inventeur: **Garbarg, Monique**
**10 rue Théodore de Banville**
**F-75017 Paris(FR)**
Inventeur: **Schunack, Walter Frei Univ. Berlin (FB Pharm.)**
**Königin-Luise-Strasse 2 + 4**
**W-1000 Berlin 33(DE)**
Inventeur: **Schwartz, Jean-Charles**
**9 Villa Seurat**
**F-75014 Paris(FR)**
Inventeur: **Lipp, Ralph Ortwin Institut für Pharmazie**
**Königin-Luise-Strasse 2 + 4**
**W-1000 Berlin 33(DE)**

(74) Mandataire: **Moncheny, Michel et al**
**c/o Cabinet Lavoix 2 Place d'Estienne d'Orves**
**F-75441 Paris Cedex 09(FR)**

**EP 0 214 058 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

SCI. PHARM., vol. 50, no. 4, 1982, pp 269-270; W. SCHUNACK: "Synthese und Wirkung chiraler Agonisten mit stereoselektiver Aktivität an Histamin-H2-Rezeptoren"

C.R. GANELLIN et al.: "Frontiers in histamine research", 1985; Pergamon Press Inc. pp 39-46, W. SCHUNAK et al.: "Chiral agonists of histamine"

CHEMICAL ABSTRACTS, vol. 84, no. 7, 16 février, p 41, réf. no. 38805p; Columbus, Ohio, US; M. BERNARDI et al.: "Protective effect of some basic aminoacids against the damage to the gastric mucosa produced by indomethacin in pylorus-ligated rats" & RIV. FARMACOL. TER. 1975, 6(3), 245-8

JOURNAL OF MEDICINAL CHEMISTRY, vol. 19, no. 7, 1976, pp 923-928; G.J. DURANT et al.: "Potential histamine H2-receptor antagonists. 3. Methylhistamines"

ARCH. PHARM., vol. 313, 1980, pp 709-714; Verlag Chemie GmbH, Weinheim, DE; G. GERHARD et al.: "Absolute Konfiguration und histaminartige Wirkung der enantiomeren alpha-Methylhistamine"

NATURE, vol. 302, no. 28, avril 1983, pp 832-837; MacMillan Journals Ltd. J.M. ARRANG et al.: "Auto-inhibition of brain histamine release mediated by a novel class (H3) of histamine receptor"

AGENTS AND ACTIONS, vol. 20, nos. 3/4, 1987, pp 239-243; Birkhäuser Verlag, Basel, CH; J.F. VAN DER WERF et al.: "H3 receptor assay in electrically-stimulated superfused slices of rat brain cortex; effects of Nalpha-alkylated histamines and impromidine analogues"

NEUROSCIENCE, vol. 15, 1985, pp 553-562, Pergamon Press Ltd., GB; J.M. ARRANG et al.: "Autoregulation of histamine release in brain by presynaptic H3-receptors"

## Description

La présente invention concerne des compositions thérapeutiques contenant des dérivés de l'histamine, notamment de 1' α-méthyl histamine et de son stéréoisomère R, de pouvoir rotatoire lévogyre et dont la configuration correspond à celle de la L-histidine et leur utilisation pour la fabrication d'un médicement inhibiteur de la synthèse d'histamine.

Les deux stéréoisomères de l' α-méthylhistamine ont été préparés antérieurement comme analogues de l'histamine mais leur étude pharmacologique n'avait permis de montrer qu'une activité histaminique très faible, voisine pour les deux stéréoisomères, sur les récepteurs $H_1$ et $H_2$, seuls connus à l'époque, (Gerhard et Schunack, Arc. Pharm. (Weinheim) 1980, 313, 709). Cette observation conduisait à écarter ce produit d'une étude pharmacologique approfondie et de toute application thérapeutique.

Or, nous venons de découvrir les propriétés agonistes puissantes et hautement stéréosélectives de l'α-méthylhistamine sur une nouvelle classe de récepteurs (récepteurs $H_3$). La stimulation des récepteurs $H_3$ induit principalement une inhibition de la synthèse et de la libération de l'histamine (ARRANG et coll., Nature, 1983, 302, 832; Neuroscience, 1985, 15, 553; Frontiers in Histamine Research, C.R. Ganellin & J.C. SCHWARTZ eds, Pergamon Press, 1985, p. 143). Ces propriétés ont été retrouvées chez quelques composés de structure proche connus dans la littérature.

L'invention concerne donc une composition pharmaceutique contenant un dérivé de l'histamine répondant à la formule

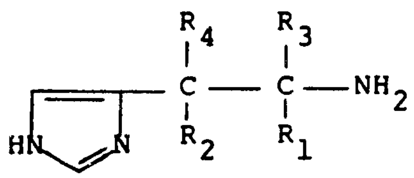

I

dans laquelle $R_1$, $R_2$ et $R_4$ désignent chacun un atome d'hydrogène ou un groupe méthyle, ou bien $R_1$ et $R_2$, pris ensemble, forment un groupe méthylène, et $R_3$ représente un atome d'hydrogène ou un groupe méthyle, sous réserve que, $R_1$, $R_2$, $R_3$ et $R_4$ ne désignent pas simultanément l'hydrogène, et un véhicule ou excipient pharmaceutiquement acceptable.

L'invention concerne plus particulièrement les exemples de composés suivants :

1. α-méthylhistamine ou 4-(2-aminopropyl)imidazole et ses stéréoisomères R(-) et S(+) ; (I : $R_1 = CH_3$ ; $R_2 = R_3 = R_4 = H$) ; cf référ. ci-dessus.
2. α,α-diméthylhistamine ou 4-(2-méthyl 2-amino propyl)-imidazole (I : $R_1 = R_3 = CH_3$ ; $R_2 = R_4 = H$) ; décrite par Schunack, Joint Meeting of the American chemical Society, Div. of Med. Chem, and the American Society for pharmacology and experimental therapeutic, Boston, USA, Aug. 18-22, 1985.
3. β-méthylhistamine ou 4-(1-méthyl 2-amino éthyl)-imidazole et ses stéréoisomeres (I : $R_2 = CH_3$ ; $R_1 = R_3 = R_4 = H$) décrite par Ganellin et al. dans J. Med. Chem. 1973, 16, 616 (sous forme racémique) et par Schunack et al. dans Frontiers in histamine research, C.R. Ganellin et J.C. Schwartz eds, Pergamon Press, 1985, p. 39 (sous forme de stéréoisomères).
4. β, β-diméthylhistamine ou 4-(1,1-diméthyl 2-amino éthyl)-imidazole (I : $R_1 = R_3 = H$ ; $R_2 = R_4 = CH_3$), décrite par Dwart et al., J. Med. chem. 1976, 19, 923.
5. 2-(4-imidazolyl)-cyclopropylamine (I : $R_1$ $R_2$ = $CH_2$ ; $R_3 = R_4 = H$), décrite par Burger et al. J. Med. Chem. 1970, 13, 33.

Etude pharmacologique.

L'effet des dérivés de l'histamine selon l'invention a été étudié sur les autorécepteurs $H_3$ contrôlant la libération de l'histamine-$^3$H induite par dépolarisation de coupes de cerveau de rat, selon le procédé décrit par Arrang. & coll. (Nature, 1983, 302, 832-837). Les dérivés se comportent comme des agonistes complets des récepteurs $H_3$ et produisent une inhibition maximale de libération, identique à celle induite par l'histamine (environ 60%). La concentration efficace 50% de l'isomère R(-) de l'α-méthylhistamine est de 2 ± 1 nM, ce qui correspond à une activité relative à l'histamine ($CE_{50}$ = 60 ± 10 nM) d'environ 3 000 % ; par contre, l'isomère S (+) a une $CE_{50}$ de 700 ± 300 nM et une activité relative à l'histamine de 9% (facteur de stéréosélectivité 350).

L'effet de l'α-méthylhistamine, comme celui de l'histamine, est reversé par divers antagonistes $H_3$, tels que l'impromidine (voir tableau 2 ci-après).

Par contre, les dérivés selon l'invention et notamment les deux isomères de l'α-méthylhistamine sont très peu actifs sur les récepteurs $H_1$ et $H_2$, sur lesquels leur activité relative à l'histamine est de 0,5 à 2% (SCHUNACK & al, Frontiers in Histamine Research, C.R. Ganellin et J.C. Schwartz eds, Pergamon Press, 1985, 39). Etant donnée la plus grande sensibilité des récepteurs $H_3$ aux agonistes, il en résulte que l'isomère R stimule ces derniers à des concentrations environ 10.000 à 100.000 fois plus faibles que celles auxquelles il stimule les récepteurs $H_1$ et $H_2$. Il constitue donc le premier agoniste hautement sélectif des récepteurs $H_3$. Les résultats sont rassemblés dans le tableau 1 ci-dessous.

TABLEAU 1

| Inhibition de la libération d'histamine-$^3$H, à partir de coupes de cortex cérébral de rat. | | | |
|---|---|---|---|
| Exemple | $CE_{50}$ (nM) | Activité relative à l'histamine (=100) | |
| | | récepteurs $H_3$ | récepteurs $H_1$/$H_2$ |
| 1 (R) | 2 ± 1 | 3000 | 0,5 - 1 |
| 1 (S) | 700 ± 300 | 9 | 0,5 - 2 |
| 2 | 20 ± 10 | 300 | <1 |
| 3(R,S) | 20 ± 5 | 300 | <1 |
| 4 | 50 ± 20 | 120 | <0,1 |
| 5 | 10 ± 5 | 600 | <1 |

TABLEAU 2

Inhibition par l'α-méthylhistamine de la libération d'histamine-$^3$H, à partir de coupes de cortex cérébral de rat.

| Conditions | Libération d'histamine-$^3$H induite par 30 mM $K^+$ (% du total) |
|---|---|
| Témoins | 16 ± 1 |
| (R)- α-méthylhistamine (100 nM) | 6 ± 1 * (-63%) |
| (R)- α-méthylhistamine (100 nM) + impromidine (100 μM) | 14 ± 2 N.S. |

* $P < 0,01$
N.S. non significatif

L'effet de l' α-méthylhistamine a aussi été étudié sur les autorécepteurs $H_3$ contrôlant la synthèse de l'histamine dans des coupes de cerveau de rat, selon ARRANG et coll., 1985, in Frontiers in Histamine Research, C.R. Ganellin et J.C. Schwartz eds, Pergamon Press, p. 143). L'α-méthylhistamine induit la

même inhibition maximale que l'histamine (environ 70%) de la stimulation par le potassium de la synthèse d'histamine-$^3$H. avec une activité relative du même ordre que dans le modèle de libération. Son effet est réversé de façon compétitive par les antagonistes $H_3$, tels que le burimamide (voir Tableau 3 ci-après).

L' $\alpha$-méthylhistamine inhibe également la synthèse d'histamine dans des coupes de cortex cérébral humain et d'hypothalamus postérieur de rat (corps mamillaires) par stimulation des récepteurs $H_3$.

**TABLEAU 3**

**Inhibition par l' $\alpha$-méthylhistamine de la stimulation de synthèse d'histamine-$^3$H, induite par le potassium dans des coupes de cortex cérébral de rat.**

| Conditions | Stimulation de synthèse d'histamine-$^3$H induite par 30 mM $K^+$ (%) |
|---|---|
| Témoins | 85 ± 4 |
| (R,S)-$\alpha$-méthylhistamine (100 nM) | 40 ± 4 * (-53%) |
| (R,S)-$\alpha$-méthylhistamine (100 nM) + burimamide (10 $\mu$M) | 90 ± 5 N.S. |

* $p < 0{,}01$
N.S. non significatif

Pour étudier in vivo l'effet de la (R)-$\alpha$-méthylhistamine sur la synthèse d'histamine, le procédé de VERDIERE et coll. (Brain Research, 1977, 129, 107) a été utilisé avec de légères modifications.

Les rats reçoivent 200 $\mu$Ci de L-histidine-$^3$H (i.v.) et l'histamine-$^3$H synthétisée est mesurée 10 minutes plus tard (voir Tableau 4 ci-après).

L'administration simultanée de (R)-$\alpha$-méthylhistamine (10 mg/kg) réduit significativement la synthèse d'histamine-$^3$H dans le cerveau et aussi dans les tissus périphériques étudiés. Cet effet étant complètement reversé par un antagoniste des récepteurs $H_3$, il résulte d'une stimulation de ces récepteurs.

Ainsi les dérivés de l'invention inhibent puissamment la libération et la synthèse d'histamine, en stimulant les récepteurs $H_3$ de manière très sélective. A ce titre, ils sont susceptibles de diminuer les transmissions histaminergiques dans le tube digestif, les systèmes nerveux, cardiovasculaire et immunitaire. Ils sont donc utilisables en thérapeutique comme médicament à effets sédatif, régulateur du sommeil, anticonvulsivant, régulateur des sécrétions hypothalamo-hypophysaires, antidépresseur, modulateur de la circulation cérébrale, etc...

Par ailleurs une inhibition de la libération des messagers de l'inflammation dans diverses affections allergiques (ex. : asthme) est attendue de la stimulation des récepteurs $H_3$ du poumon, par exemple.

En gastro entérologie, l'inhibition de libération d'histamine gastrique est susceptible d'exercer des effets anti-secrétoire et anti-ulcéreux. Une modification de libération des messagers des réponses immunitaires est susceptible de moduler ces dernières.

En raison de ces propriétés originales et inattendues les dérivés de l'invention et en particulier 1' $\alpha$-méthylhistamine , principalement sous la forme de son stéréoisomère R,peuvent constituer le principe actif de compositions pharmaceutiques comportant également un véhicule ou excipient pharmaceutiquement acceptable. Leur mode d'action, leurs divers effets pharmacologiques et leur faible toxicité chez l'animal

laissent prévoir des applications en médecine humaine et vétérinaire à des doses de l'ordre de 0,1 à 10 mg/kg, notamment par voie orale et parentérale.

Ils peuvent être présentés notamment sous forme de comprimés, dragées, gélules, aérosols, solutés injectables à l'exclusion de solutions comme indiquées dans des revendications, ou suppositoires.

TABLEAU 4

| Inhibition par la (R) α-méthylhistamine de la synthèse d'histamine-$^3$H chez le rat in vivo. | | | |
|---|---|---|---|
| Tissu | Histamine-$^3$H (% de la radio-activité totale x $10^{-3}$) | | Variation (%) |
| | Témoins | Traités | |
| Cortex cérébral | 96 ± 6 | 53 ± 6 | - 45 ** |
| Hypothalamus | 272 ± 11 | 185 ± 32 | - 32 ** |
| Poumons | 138 ± 10 | 94 ± 10 | - 32 * |
| Rate | 60 ± 8 | 42 ± 5 | - 30 * |
| Reins | 53 ± 4 | 37 ± 5 | - 30 * |

* p <0,05
** p <0,01

Les animaux (6-13) ont reçu une injection intraveineuse de 200 µCi de L-histidine-$^3$H seule (témoins) ou avec de la (R) α-méthylhistamine -10 mg/kg (traités) et ont été sacrifiés 10 minutes plus tard.

L'histamine-$^3$H extraite des tissus par $HClO_4$ a été isolée.

## Revendications

**1.** Composition pharmaceutique contenant un dérivé de l'histamine repondant à la formule

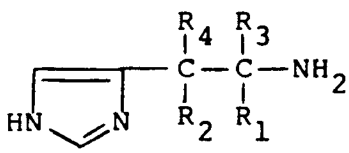

I

dans laquelle $R_1$, $R_2$ et $R_4$ désignent chacun un atome d'hydrogène ou un groupe méthyle, ou bien $R_1$ et $R_2$, pris ensemble, forment un groupe méthylène et $R_3$ désigne un atome d'hydrogène ou un groupe méthyle, sous réserve que $R_1$, $R_2$, $R_3$ et $R_4$ ne désignent pas simultanément l'hydrogène, et un véhicule ou excipient pharmaceutiquement acceptable à l' exclusion des solutions parenterales contenant 2-(µ-imidatolyle)cyclopropylamine ou des solutions aqueuses contentant α-méthylhistamine.

**2.** Composition pharmaceutique selon la revendication 1 contenant de l'α-méthylhistamine et un véhicule ou excipient pharmaceutiquement acceptable.

**3.** Composition pharmaceutique selon les revendications 1, et 2, contenant de la (R)-α-méthylhistamine et un véhicule ou excipient pharmaceutiquement acceptable.

**4.** Médicament, selon les revendications 1,-3, notamment à effet stimulateur des récepteurs $H_3$, contenant de la α-méthylhistamine ou son isomère R.

**5.** Utilisation d'un dérivé de formule

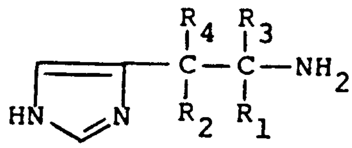

I

dans laquelle $R_1$, $R_2$ et $R_4$ désignent chacun un atome d'hydrogène ou un groupe méthyle, ou bien $R_1$ et $R_2$, pris ensemble, forment un groupe méthylène et $R_3$ désigne un atome d'hydrogène ou un groupe méthyle sous réserve que $R_1$, $R_2$, $R_3$ et $R_4$ ne désignent pas simultanément l'hydrogène, pour la fabrication d'un médicament inhibiteur de la synthèse de l'histamine, notamment à effets sédatif, régulateur du sommeil, anticonvulsivant, antidépresseur, antiallergique, anti-secrétoire ou anti-ulcéreux.

**Claims**

**1.** A pharmaceutical composition containing a histamine derivative of formula

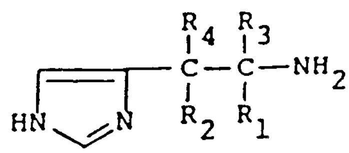

I

in which $R_1$, $R_2$ and $R_4$ each denotes a hydrogen atom or a methyl group, or $R_1$ and $R_2$ taken together form a methylene group and $R_3$ denotes a hydrogen atom or a methyl group, provided that $R_1$, $R_2$, $R_3$ and $R_4$ do not simultaneously denote hydrogen, and a pharmaceutically acceptable vehicle or excipient, excluding parenteral solutions containing 2(4-imidazolyl)cyclopropylamine or aqueous solutions containing $\alpha$-methylhistamine.

**2.** A pharmaceutical composition according to Claim 1, containing $\alpha$-methylhistamine and a pharmaceutically acceptable vehicle or excipient.

**3.** A pharmaceutical composition according to Claims 1 and 2, containing (*R*)-$\alpha$-methylhistamine and a pharmaceutically acceptable vehicle or excipient.

**4.** A medicament according to Claims 1-3, especially with stimulant effect on the $H_3$-receptors, containing $\alpha$-methylhistamine or its *R*-isomer.

**5.** The use of a derivative of formula

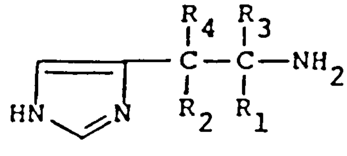

I

in which $R_1$, $R_2$ and $R_4$ each denotes a hydrogen atom or a methyl group, or $R_1$ and $R_2$ taken together form a methylene group and $R_3$ denotes a hydrogen atom or a methyl group, provided that $R_1$, $R_2$, $R_3$ and $R_4$ do not simultaneously denote hydrogen, for the production of a medicament inhibiting histamine synthesis, especially with sedative, sleep-regulating, antiepileptic, antidepressant, antiallergic, antisecretory or antiulcer effects.

**Patentansprüche**

**1.** Pharmazeutische Zusamensetzung enthaltend ein Histamin-Derivat gemäß der Formel

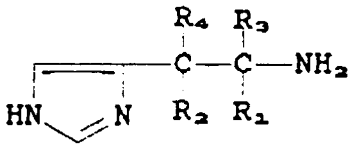

I

in welcher $R_1$, $R_2$ und $R_4$ jeweils ein Wasserstoff-Atom oder eine Methyl-Gruppe bedeuten oder aber $R_1$ und $R_2$ zusammengenommen eine Methylen-Gruppe bilden und $R_3$ ein Wasserstoff-Atom oder eine Methyl-Gruppe bedeutet, mit der Maßgabe, daß $R_1$, $R_2$, $R_3$ und $R_4$ nicht gleichzeitig Wasserstoff bedeuten, und einen pharmazeutisch annehmbaren Träger oder Hilfsstoff mit Ausnahme 2-(4-Imidazolyl)cyclopropylamin enthaltender parenteraler Lösungen und $\alpha$-Methylhistamin enthaltender wäßriger Lösungen.

**2.** Pharmazeutische Zusammensetzung gemäß Anspruch 1, enthaltend $\alpha$-Methylhistamin und einen pharmazeutisch annehmbaren Träger oder Hilfsstoff.

**3.** Pharmazeutische Zusammensetzung gemäß einem der Ansprüche 1 oder 2, enthaltend (R)-$\alpha$-Methylhistamin und einen pharmazeutisch annehmbaren Träger oder Hilfsstoff.

**4.** Arzneimittel gemäß Anspruch 1 bis 3, insbesondere mit $H_3$-Rezeptoren stimulierender Wirkung, enthaltend $\alpha$-Methylhistamin oder dessen (R)-Isomer.

**5.** Verwendung eines Derivates der Formel

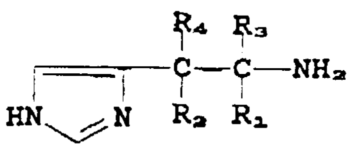

I

in welcher $R_1$, $R_2$ und $R_3$ jeweils ein Wasserstoff-Atom oder eine Methyl-Gruppe bedeuten oder aber $R_1$ und $R_2$ zusammengenommen eine Methylen-Gruppe bilden und $R_3$ ein Wasserstoff-Atom oder eine Methyl-Gruppe bedeutet, mit der Maßgabe, daß $R_1$, $R_2$, $R_3$ und $R_4$ nicht gleichzeitig Wasserstoff bedeuten, zur Herstellung eines die Histamin-Synthese hemmenden Arzneimittels mit insbesondere sedierender, schlafregulierender, antikonvulsiver, antidepressiver, antiallergischer, antisekretorischer oder antiulcus-Wirkung.